# EUROPÄISCHE PATENTSCHRIFT

(11) **EP 1 255 557 B1**
(45) Veröffentlichungstag und Bekanntmachung des Hinweises auf die Patenterteilung: **12.11.2003**
(21) Anmeldenummer: 01901008.1
(22) Anmeldetag: 10.01.2001
(51) Int. Cl.: A61K 38/11, A61K 9/08, A61K 47/12

(54) **STABILE, NASAL, ORAL ODER SUBLINGUAL ANWENDBARE PHARMAZEUTISCHE ZUBEREITUNG ENTHALTEND DESMOPRESSIN**
STABLE, NASALLY, ORALLY OR SUBLINGUALLY APPLICABLE PHARMACEUTICAL PREPARATION CONTAINING DESMOPRESSIN
PREPARATION PHARMACEUTIQUE STABLE, POUVANT ETRE UTILISEE PAR VOIE NASALE, ORALE OU SUBLINGUALE CONTENANT DE LA DESMOPRESSINE

(30) Priorität: 16.02.2000 AT 2332000
(43) Veröffentlichungstag der Anmeldung: 13.11.2002
(73) Patentinhaber: Gebro Pharma GmbH, 6391 Fieberbrunn (AT)
(72) Erfinder: SCHEIDL, Helmut, A-6391 Fieberbrunn (AT); HANTICH, Gerhard, A-6370 Kitzbühel (AT); HESSE, Ernst, A-6391 Fieberbrunn (AT); ZAPF, Thomas, D-53175 Bonn (Deutschland) (DE)
(74) Vertreter: Wildhack, Helmut, Dr. Dipl.-Ing.
(86) Internationale Anmeldenummer: AT0100007
(87) Internationale Veröffentlichungsnummer: WO01060394

(56) Entgegenhaltungen:
- EP-A- 0 517 211
- EP-A- 0 702 958
- WO-A-95/01185
- US-A- 5 674 850
- DATABASE WPI Week 198826 Derwent Publications Ltd., London, GB; AN 1988-178953 [28] XP002145884 & JP 63 115821 A (TEIJIN LTD.,JP) 20. Mai 1988 (1988-05-20)
- DATABASE CHEMABS [Online] CHEMICAL ABSTRACTS SERVICE, COLUMBUS, OHIO, US; NAKAKURA, MASASHI ET AL: "pharmaceuticals containing electric antioxidants as stabilizers for iontophoresis" retrieved from STN Database accession no. 121:18095 CA XP002146871 & JP 06 040948 A (KYOWA HAKKO KOGYO KK, JAPAN) 15. Februar 1994 (1994-02-15)

## Beschreibung

Die Erfindung bezieht sich auf eine stabile, nasal, oral oder sublingual anwendbare pharmazeutische Zubereitung zur Anwendung am Patienten, in Form einer flüssigen, insbesondere wässerigen, Lösung von Desmopressin als aktivem Wirkstoff, wobei diese flüssige Lösung ein Osmotikum und einen Puffer enthält, welcher den pH-Wert im Bereich 4 bis 6, vorzugsweise bei etwa 5, hält.

Desmopressin (1-Deamino-8-D-Arginin-Vasopressin) ist ein Peptid-Hormon, welches therapeutisch stark wirksam ist. In flüssigen pharmazeutischen Zubereitungen liegt es daher in der Regel in geringer Konzentration vor. Um die Wirksamkeit der Zubereitung zu gewährleisten, muss eine Stabilisierung erfolgen, um den chemischen und mikrobiellen Abbau zu minimieren. Hiefür wurde bereits vorgeschlagen (US 5 482 931 A bzw. WO 95/01185), Benzalkoniumchlorid als Konservierungsmittel und einen geeigneten Puffer einzusetzen, welcher den pH-Wert der wässerigen Zusammensetzung auf 4 bis 6, vorzugsweise bei etwa 5, hält. Die beste Stabilisierung von Desmopressin lässt sich hierbei mit Acetat als Puffer erreichen. Dies befriedigt jedoch in der Praxis nicht, da Essigsäure einen unangenehmen Geruch aufweist. In den erwähnten Literaturstellen ist daher auch ein Citrat-Phosphat-Puffersystem vorgeschlagen, stets in Kombination mit Benzalkoniumchlorid als Konservierungsmittel, dem auch zugeschrieben wird, dass es die Adsorption an Gefäßwänden verhindert.

Die Erfindung setzt sich zur Aufgabe, die Stabilisierung des Wirkstoffes Desmopressin in einer pharmazeutischen Zubereitung der eingangs geschilderten Art weiter zu verbessern und dies unabhängig von der Verwendung bzw. Art eines Konservierungsmittels.

Untersuchungen haben überraschenderweise gezeigt, dass sich durch die Verwendung von Apfelsäure als Puffer die angestrebten Vorteile problemlos erreicht werden, ohne dass Nachteile anderer Art in Kauf genommen werden müssen. Eine pharmazeutische Zubereitung der erfindungsgemäßen Art enthält daher Desmopressin als Wirkstoff, insbesondere in niedriger Konzentration, weiters Apfelsäure zur Stabilisierung des Desmopressins und als Puffer zur Einstellung des pH-Wert auf den Bereich 4 bis 6, vorzugsweise bei etwa 5, und einen geeigneten Zusatz als Osmotikum. Die Apfelsäure hat im Zusammenhang mit der vorliegenden Erfindung somit eine Doppelfunktion: einerseits bildet sie den Puffer zur Einstellung des pH-Wertes, anderseits sichert sie die Stabilisierung des Desmopressins.

Die pharmazeutische Anwendung der erfindungsgemäßen Zubereitung liegt hauptsächlich in der Behandlung von antidiuretischen Störungen, insbesondere Enuresis nocturna und Diabetes insipidus. Weiters ist die Behandlung von Blutungskrankheiten, wie z.B. Hämophilie A, Willebrand-Jürgens-Syndrom und postoperativen Blutungen, möglich.

Es genügt in der Regel, den Apfelsäure-Puffer in niedriger Konzentration, vorzugsweise im Bereich 1 bis 5 mM, insbesondere bei etwa 2,5 mM, einzusetzen. Die Apfelsäure kann hierbei als Racemat vorliegen, was finanziell günstig ist, es können jedoch auch die D- bzw. L-Form eingesetzt werden, auch in Kombination untereinander bzw. mit dem Racemat.

Als Osmotikum eignet sich Natriumchlorid in bekannter Weise.

Zusätzlich zur Apfelsäure können weitere Puffer eingesetzt werden, z.B. Acetat/Essigsäure, ohne dass darunter die Vorteile der Apfelsäure leiden.

Von besonderem Vorteil ist es, dass die erfindungsgemäße Zubereitung freigehalten werden kann von Konservierungsmitteln, ohne dass dadurch die Stabilisierung des Wirkstoffes Desmopressin leidet. Es kann daher die Verkeimung der Desmopressinlösung im Apfelsäuresystem durch aseptische Abfüllung und/oder durch Zusatz von antimikrobiellen Substanzen verhindert werden.

Die erfindungsgemäße Zubereitung eignet sich für die nasale oder orale oder sublinguale Anwendung.

Weitere Kennzeichen und Vorteile der Erfindung ergeben sich aus der Beschreibung der folgenden Ausführungsbeispiele bzw. der Vergleichsversuche:

Hierbei wurden folgende Lösungen von Desmopressin-Acetat als Rezepturen verwendet:

| Rezeptur-Nr. | Desmopressinacetat in mg/ml | Konservierungsmittel in mg/ml | Puffer [Konz. in mM] |
|---|---|---|---|
| 1 | 0,100 | Benz. 0,10 | DL-Apfelsäure [2,5] |
| 2 | 0,10 | Benz. 0,10 | Apfelsäure [25] |
| 3 | 0,10 | Benz. 0,10 | Citronensäure und Na₂HPO₄ [zusammen 25] |
| 4 | 0,10 | Benz. 0,10 | NaH₂PO₄[19] pH = 1,0 |
| 5 | 0,11 | Benz. 0,10 | NaH₂PO₄[19] pH = 2,0 |
| 6 | 0,10 | Benz. 0,10 | NaH₂PO₄[19] pH = 3,0 |
| 7 | 0,11 | Benz. 0,10 | NaH₂PO₄[19] pH : 4,0 |
| 8 | 0,10 | Benz. 0,10 | KH₂PO₄ und Na₂HPO₄ [zusammen 19] pH = 5,0 |
| 9 | 0,10 | Benz. 0,13 | Citronensäure [60] pH = 6,0 |
| 10 | 0,10 | Benz. 0,13 | KH₂PO₄ und Na₂HPO₄ [zusammen 67] pH = 7,0 |
| 11 | 0,10 | Benz. 0,10 | L-Apfels [2,5] |
| 12 | 0,10 | Benz. 0,10 | D-Apfels [2,5] |
| 13 | 0,10 | Benz. 0,10 | Apfels [2,5] |
| 14 | 0,10 | Benz. 0,10 | Apfels/NaAc: [2,5] |
| 15 | 0,10 | - | Apfels [2,5] |
| 16 | 0,02 | Benz. 0,10 | Apfels [2,5] |
| 17 | 2,00 | Benz. 0,10 | Apfels [2,5] |
| 18 | 0,10 | Benz. 0,10 | Apfels [1,0] |
| 19 | 0,10 | Benz. 0,10 | Apfels [5,0] |
| 20 | 0,10 | Benz. 0,05 | Apfels [2,5] |
| 21 | 0,10 | Benz. 0,20 | Apfels [2,5] |
| 22 | 0,10 | p-Hydroxyb. 2,0 | Apfels [2,5] |
| 23 | 0,10 | - | 80 % Apfels [2,5] und 20 % Cit/PO₄³⁻ [25] |
| 24 | 0,10 | - | 60 % Apfels [2,5] und 40 % Cit/PO₄³⁻ [25] |
| 25 | 0,10 | - | 50 % Apfels [2,5] und 50 % Cit/PO₄³⁻ [25] |
| 26 | 0,10 | - | 40 % Apfels [2,5] und 60 % Cit/PO₄³⁻ [25] |
| 27 | 0,10 | - | 20 % Apfels [2,5] und 80% Cit/PO₄³⁻ [25] |
| Hierin und in den folgenden Tabellen gelten folgende Abkürzungen: Apfels - Apfelsäre Cit./PO₄³⁻ - Citrat-Phosphat Ac - Acetat HAc - Essigsäure Benz - Benzalkoniumchlorid NH₄Ac - Ammoniumacetat p-Hydroxyb. - p-Hydroxybenzoesäuremethylester mM - milliMol/Liter | | | |

Für die Herstellung der in den folgenden Versuchen verwendeten Desmopressin-Lösungen (jeweils 1 l) wurde generell folgendes Verfahren eingehalten:
a) Einwaage von 989,15 g Aqua des. ad. inj. in ein 1 l Becherglas.
b) Davon ca. 30 g Aqua dest. in ein Becherglas zum Nachspülen geben.
c) In das restliche Aqua dest. von a) wurden unter Rühren mit dem Magnetrührer 9,115 g Natriumchlorid Ph.Eur. reinst und der verwendete Puffer (im Falle von Apfelsäure 0,335 g) gelöst. Die Einwaagegefäße wurden mit jeweils ca. 5 g Aqua dest. von b) gespült.
d) Unter Rühren wurde die jeweils verwendete Menge an Desmopressinacetat 100 % (istbezogene Einwaage) zugegeben und das Einwaagegefäß zweimal mit ca. 5 g Aqua dest. gespült.
e) Gegebenenfalls wurde die jeweils verwendete Menge an Konservierungsmitteln (zumeist Benzalkoniumchlorid 100%) (istbezogene Einwaage) zugegeben und das Einwaagegefäß zweimal mit ca. 5 g Aqua dest, gespült, anschließend ca. ½ Stunde gerührt.
f) Der pH Wert wurde mit ca. 4,2 ml 1 normale NaOH-Lösung auf den für die jeweilige Rezeptur gültigen pH-Wert (zumeist 5,0 ± 0,2) eingestellt.
g) 1003,0 g Endlösung entsprechen 1000 ml.
h) Die Sterilfiltration der Endlösung erfolgte mit einem Millipak-Sterilfilter.

Die jeweils verwendeten Substanzen wurden von folgenden Herstellern bezogen:

| Substanz | Hersteller |
|---|---|
| Desmopressinacetat | UCB Belgien |
| Benzalkoniumchlorid | Ferrosan |
| p-Hydroxybenzoesäuremethylester | Merck Darmstadt |
| NaCI | Österr. Salinen AG bzw. Merck |
| HCI | 1 N. (Art.Nr. 10448) Merck Darmstadt |
| NaOH | Plätzchen, Merck Darmstadt |
| DL-Apfelsäure | Merck Darmstadt |
| Essigsäure | 100 % reinst., Merck Darmstadt |
| Millipak-Filter 0,22 µm (Durapore®: PVDF) | Millipore |

### Vergleichende Untersuchung der Stabilität von Desmopressin:

Hiefür wurde einerseits eine Lösung nach dem Stand der Technik verwendet (Rezeptur Nr. 3), anderseits eine erfindungsgemäße Rezeptur (Rezeptur Nr. 1). Die beiden Rezepturen wurden jeweils bei 25°C und 50°C über einen Zeitraum von jeweils 10 Monaten gelagert und danach im Hinblick auf den Gehalt an den Abbauprodukten G1, G2, G3 und G4 analysiert. Hiebei handelt es sich um folgende Abbauprodukte:
G1 - 5-Asparaginsäuredesmopressin
G2 - 4-Glutaminsäuredesmopressin
G3 - 9-Glycindesmopressin
G4 - Isomeres von 5-Asparaginsäuredesmopressin.

Die in der folgenden Tabelle angegebenen Werte geben die Abbauprodukte, bezogen auf Desmopressin, an (in % A/A, d.h. Flächenprozente bezogen auf den Wirkstoff):

| Rezeptur Nr. | 10 Monate / 50°C | | | | 10 Monate /25°C | | | |
|---|---|---|---|---|---|---|---|---|
| | G4 | G3 | G1 | G2 | G4 | G3 | G1 | G2 |
| 3 | 5,27 | 1,85 | 1,16 | 2,44 | 0,26 | 0,18 | 0,07 | 0,21 |
| 1 | 3,45 | 1,19 | 0,95 | 1,48 | 0,14 | 0,11 | 0,05 | 0,13 |

Wie aus dem Vergleich der gewonnen Werte der vier Abbauprodukte des Desmopressins ersichtlich ist, ergibt das Apfelsäure-Puffersystem eine wesentlich höhere Stabilität des Wirkstoffes Desmopressin als das Citrat-Phosphat-Puffersystem, und dies sowohl nach Lagerung der Lösungen bei Raumtemperatur als auch unter Stressbedingungen.

### Desmopressin-Stabilität im Apfelsäure-Puffersystem:

Durch Vorversuche wurde festgestellt, dass Desmopressin im Bereich von pH = 5,0 am stabilsten ist. Für diese Vorversuche wurden Desmopressin-Zubereitungen untersucht, die unterschiedliche pH-Werte aufwiesen, u.zw. die pH-Werte 1,0; 2,0; 3,0; 4,0; 5,0; 6,0 und 7,0. Diese Lösungen wurden in Glaskolben 6 Wochen lang bei 50°C gelagert und dann in üblicher Weise mittels analytischer Säulen analysiert. Nach 6 Wochen waren die pH-Werte der sieben Lösungen unverändert. Die Untersuchungen auf die Gehaltswerte von Desmopressin und die Flächensumme der früher erwähnten Abbauprodukte G1, G2, G3 und G4 ergaben, dass Desmopressin im Bereich von pH = 5,0 am stabilsten ist.

Es wurden daher isotone Desmopressin-Lösungen gemäß den Rezepturen 1 und 2 untersucht, welche Rezepturen somit Apfelsäure als Puffer in der Konzentration 2,5 mM bzw. 25 mM aufwiesen. Der pH-Wert beider Zubereitungen war 5,0. Die beiden Desmopressin-Lösungen wurden zwei Monate in Glasflaschen bei 40°C gelagert. Danach erfolgte die Bestimmung von Desmopressin und der Abbauprodukte 9-Glycindesmopressin (G3) und 5-Asparaginsäuredesmopressin (G1).

Das Ergebnis ist in der nachfolgenden Tabelle als Verhältnis Abbauprodukt zu Desmopressin ausgedrückt, normiert auf die Rezeptur mit dem kleinsten Masseverhältnis.

| Rezeptur Nr. | Apfelsäurekonzentrat/mM | G1 | G3 |
|---|---|---|---|
| 1 | 2,5 | 1,0 | 1,0 |
| 2 | 25,0 | 1,9 | 1,3 |

Wie an den Daten zu sehen ist, zeigt Desmopressin im verdünnteren Apfelsäurepuffer in überraschenderweise eine wesentlich geringere Menge an Abbauprodukten, d.h. eine höhere Stabilität.

### Desmopressin-Stabilität in Abhängigkeit der chiralen Form der Apfelsäure:

Es wurden die Rezepturen Nr. 1, 11 und 12 hergestellt. Diese Lösungen wurden nach 2 und 4 Wochen Lagerung bei 65°C in Glasgefäßen der hydrolytischen Klasse 1 im Hinblick auf den Desmopressingehalt und die Menge an Abbauprodukten analysiert.

Es ergaben sich folgende Resultate:

| Rezeptur Nr. | Apfelsäuretypus | Desmopressinkonzentration in µg/ml nach | | Summe der Abbauprodukte in % A/A nach | |
|---|---|---|---|---|---|
| | | 2 Wochen | 4 Wochen | 2 Wochen | 4 Wochen |
| 1 | DL | 94,9 | 93,2 | < 0,2 | 0,72 |
| 11 | L | 94,6 | 93,4 | 0,2 | 0,65 |
| 12 | D | 95,1 | 93,2 | <0,2 | 0,54 |

Die Summe der Abbauprodukte beinhaltet:
5-Asparaginsäuredesmopressin,
4-Glutaminsäuredesmopressin,
9-Glycindesmopressin,
Isomeres 5-Asparaginsäuredesmopressin.

Wie den Daten zu entnehmen ist, spielt die chirale Form der Apfelsäure keine Rolle für die Stabilität des Desmopressins.

### Einfluss des Konservierungsmittels Benzalkoniumchlorid auf die Stabilität von Desmopressin:

Es wurden die Rezepturen Nr. 13, 14 und 15 einander untersuchungsmäßig gegenübergestellt, wobei in Rezeptur 14 der DL-Apfelsäurepuffer um 20 % mol/mol reduziert und entsprechend durch Acetatpuffer ersetzt wurde. Hierbei wurde wie folgt vorgegangen:

Über 7 Wochen wurden die Lösungen bei 65°C gelagert und in dieser Zeit (nach 1, 2, 3, 5, 7 Wochen) im Hinblick auf den Desmopressingehalt und den Gehalt an Abbauprodukten (G1, G2, G3, G4) analysiert.

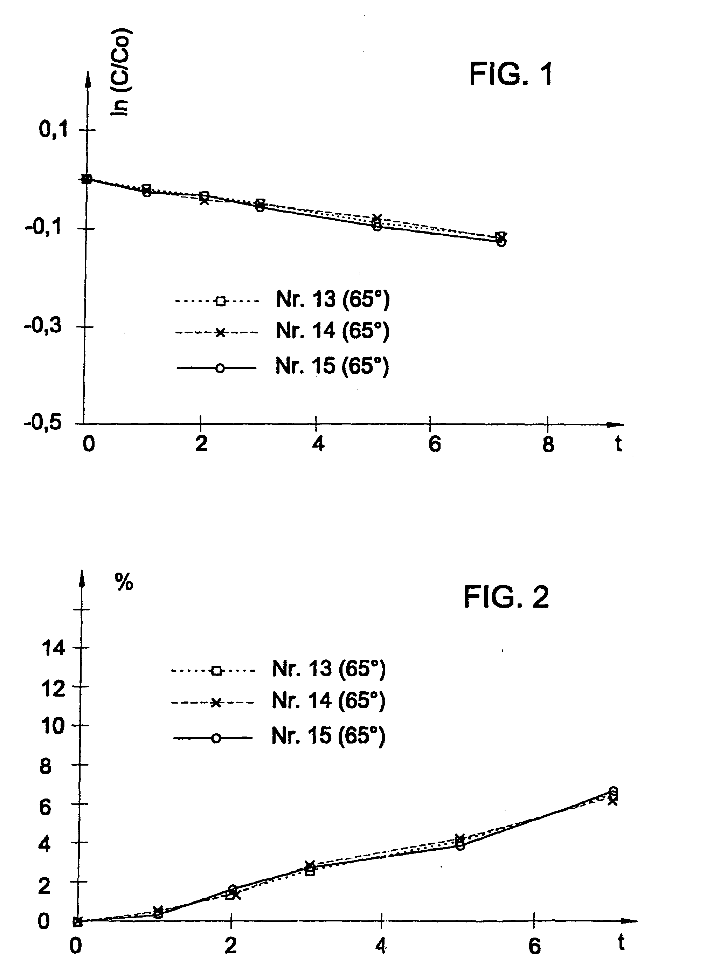

Fig. 1 zeigt die Abnahme des Desmopressingehaltes, Fig. 2 die Zunahme der Summe der Abbauprodukte G1 bis G4, wobei in beiden Figuren auf der x-Achse die Zeit t in Wochen aufgetragen ist. Auf der y-Achse ist in Fig. 1 der Wert In c/cₒ aufgetragen, in Fig. 2 die Abbauprodukte in %, bezogen auf Desmopressin.

Die folgende Tabelle zeigt die errechneten Geschwindigkeitskonstanten, wobei gilt:
In c/cₒ = -kt;
t = 7 Wochen = 4233600 s;
T = 65°C

| Rezeptur Nr. | Zusammensetzung | k 65 C/s-1 • 10-8 |
|---|---|---|
| 13 | Apfelsäure mit Benz. | 2,98 |
| 14 | Apfelsäure/Essigsäure + Benz. | 2,93 |
| 15 | Apfelsäure ohne Benz. | 3,07 |

Aus den obigen Ergebnissen lässt sich der Schluss ziehen, dass die verbesserte Stabilisierung von Desmopressin auf dem Apfelsäurepuffer und nicht auf der Gegenwart von Benzalkoniumchlorid beruht, denn der Unterschied der Ergebnisse in den Rezepturen 13, 14 und 15 ist so gering, dass er im experimentellen Fehlerbereich liegt und daher ohne Bedeutung ist.

Ferner lässt sich aus den obigen Ergebnissen der Schluss ziehen, dass auch in Gegenwart einer weiteren Puffersubstanz (wie z.B. Acetat) die Apfelsäure das Desmopressin besser stabilisiert als dies bisher übliche Puffersysteme konnten.

### Untersuchung der Stabilität von Desmopressin im Apfelsäure-Puffersystem im Vergleich zum Citrat-Phosphat-Puffersystem:

Hierbei wurde eine Lösung nach der Rezeptur Nr. 1 verglichen mit einer Rezeptur Nr. 3, wobei eine Serie von Mischungen dieser beiden Rezepturen mit unterschiedlichen Mischungsverhältnissen hergestellt wurde. Diese Mischungen wurden 4 Wochen bei 65°C gelagert und nach 2 und 4 Wochen Lagerung im Hinblick auf den Gehalt an Desmopressin und dessen Abbauprodukte (G1 bis G4) analysiert.

Die Ergebnisse sind in den beiden folgenden Tabellen zusammengefasst, wobei die Werte für die Nebenpeaks in % (A/A), bezogen auf Desmopressin, die Werte für Desmopressin in µg/ml (entspr. % vom Sollwert).

Die Bedeutungen für die Abbauprodukte G1 bis G4 entsprechen den früher angegebenen Bedeutungen.
Werte nach 2 Wochen bei 65°C:

| Mischungsverhältnis DL-Apfelsäure (Rez. Nr. 1) zu Citrat/Phosphat (Rez. 3) | G3 | G1 | G2 | G4 | Unbekannte Nebenpeaks | Summe aller Nps | Desmopressin |
|---|---|---|---|---|---|---|---|
| 100 : 0 | 0,31 | 0,29 | 0,41 | 0,85 | 0 | 1,86 | 94,85 |
| 80 : 20 | 0,4 | 0,41 | 0,51 | 1,28 | 0,41 | 3,01 | 94,39 |
| 60 : 40 | 0,46 | 0,44 | 0,57 | 1,43 | 0,51 | 3,41 | 93,3 |
| 50 : 50 | 0,46 | 0,43 | 0,58 | 1,42 | 0,54 | 3,43 | 93,28 |
| 40 : 60 | 0,41 | 0,4 | 0,53 | 1,29 | 0,32 | 2,95 | 93,24 |
| 20 : 80 | 0,49 | 0,45 | 0,64 | 1,47 | 0,65 | 3,7 | 92,9 |
| 0 : 100 | 0,5 | 0,43 | 0,67 | 1,43 | 0,65 | 3,68 | 92,28 |

Werte nach 4 Wochen bei 65°C:

| Mischungsverhältnis DL-Apfelsäure (Rez. Nr. 1) zu Citrat/Phosphat (Rez. 3) | G3 | G1 | G2 | G4 | Unbekannte Nebenpeaks | Summe aller Nps | Desmopressin |
|---|---|---|---|---|---|---|---|
| 100 : 0 | 0,64 | 0,5 | 0,64 | 1,62 | 0,72 | 4,12 | 93,21 |
| 80 : 20 | 0,66 | 0,62 | 0,73 | 2,11 | 1,23 | 5,35 | 92,20 |
| 60 : 40 | 0,75 | 0,7 | 0,85 | 2.37 | 1,31 | 5,98 | 91,26 |
| 50 : 50 | 0,69 | 0,66 | 0,83 | 2,18 | 1,34 | 5,7 | 91,43 |
| 40 : 60 | 0,64 | 0,61 | 0,78 | 2,07 | 1,49 | 5,59 | 91,53 |
| 20 : 80 | 0,79 | 0,72 | 0,95 | 2,37 | 1,49 | 6,32 | 89,97 |
| 0 : 100 | 0,84 | 0,74 | 0,99 | 2,34 | 1,69 | 6,6 | 89,74 |

Aus den obigen Tabellen ist ersichtlich, dass es durch Zumischung von Apfelsäurepuffer zum Citrat/Phosphatpuffer zu einer zunehmenden Stabilisierung von Desmopressin kommt. Dies zeigt sich am höheren Desmopressingehalt, wie auch am geringeren Gehalt der Desmopressin-Abbauprodukte nach 2 bzw. 4 Wochen Lagerung bei 65°C. Somit zeigt auch dieser Versuch die bessere Eignung des Apfelsäurepuffers im Vergleich zu bekannten Systemen, um Desmopressin in Lösung chemisch zu stabilisieren.

### Untersuchung der Stabilität von Desmopressin bei unterschiedlichen Konzentrationen:

Um verschiedene Konzentrationen am Wirkstoff (Desmopressin) zu testen, wurde die Rezeptur Nr. 1 dahingehend abgewandelt, dass der Gehalt von 0,100 mg/ml an Desmopressinacetat ersetzt wurde durch einen Gehalt von 2,00 mg/ml (Rezeptur 17) bzw. durch einen Gehalt von 0,02 mg/ml (Rezeptur 16). Die so hergestellten Lösungen wurden 4 Wochen bei 65°C gelagert und jeweils nach 2 und 4 Wochen im Hinblick auf den Gehalt an Desmopressin und dessen Abbauprodukten (G1 bis G4) - ausgedrückt als Flächensumme - analysiert.

| Rezeptur Nr. | Desmopressinacetat (% vom Sollwert) nach | | Summe der Abbauprodukte G1 - G4 (% A/A, bezogen auf Desmopressinacetat) nach | |
|---|---|---|---|---|
| | 2 Wochen | 4 Wochen | 2 Wochen | 4 Wochen |
| 17 | 96,6 | 91,6 | 1,76 | 4,03 |
| 16 | 95,9 | 92,7 | 7,9 | 8,7 |

Wie die Ergebnisse zeigen, sind die Rezepturen stabil genug, sodass man den Desmopressinacetatgehalt in einem gewissen Rahmen variieren kann, ohne dass die Stabilität des Desmopressins darunter wesentlich leidet.

### Untersuchung der Stabilität von Desmopressin in verschieden konzentrierten Apfelsäurelösungen:

Um den Einfluss der Konzentration des Puffers (Apfelsäure) zu testen, wurde die Rezeptur Nr. 1 dahingehend abgewandelt, dass die Konzentration des Apfelsäuregehaltes (2,5 mM) der Rezeptur Nr. 1 ersetzt wurde durch eine Konzentration von 1,0 mM (Rezeptur Nr. 18) bzw. durch eine Konzentration von 5,0 mM (Rezeptur Nr. 19).

Die so hergestellten Lösungen wurden 4 Wochen bei 65°C gelagert und jeweils nach 2 und 4 Wochen im Hinblick auf den Gehalt an Desmopressin und dessen Abbauprodukte (G1 bis G4) - ausgedrückt als Flächensumme - analysiert.

Es ergaben sich folgende Werte:

| Rezeptur Nr. | Desmopressinacetat (% vom Sollwert) nach | | Summe der Abbauprodukte G1 - G4 (% A/A, bezogen auf Desmopressinacetat) nach | |
|---|---|---|---|---|
| | 2 Wochen | 4 Wochen | 2 Wochen | 4 Wochen |
| 18 | 97,3 | 95,3 | 1,86 | 3,9 |
| 19 | 96,9 | 95,4 | 1,93 | 4,27 |

Wie die Ergebnisse zeigen, ist die Rezeptur stabil genug, dass man die Konzentration des Apfelsäurepuffers in einem gewissen Rahmen variieren kann, ohne an Stabilität des Desmopressingehaltes wesentlich zu verlieren.

### Untersuchung der Stabilität von Desmopressin in Anwesenheit unterschiedlicher Konzentrationen von Benzalkoniumchlorid:

Um den Einfluss der Konzentration des Konservierungsmittels Benzalkoniumchlorid zu testen, wurde die Rezeptur Nr. 1 dahingehend abgewandelt, dass der Gehalt von 0,100 mg/ml Benzalkoniumchlorid abgewandelt wurde durch einen Gehalt von 0,20 mg/ml (Rezeptur Nr. 21) bzw. durch einen Gehalt von 0,05 mg/ml (Rezeptur Nr. 20).

Die so hergestellten Lösungen wurden 4 Wochen bei 65°C gelagert und jeweils nach 2 und 4 Wochen im Hinblick auf den Gehalt an Desmopressin und dessen Abbauprodukten (G1 bis G4) - ausgedrückt als Flächensumme - analysiert.

Es ergaben sich folgende Werte:

| Rezeptur Nr. | Desmopressinacetat (% vom Sollwert) nach | | Summe der Abbauprodukte G1 - G4 (% A/A, bezogen auf Desmopressinacetat) nach | |
|---|---|---|---|---|
| | 2 Wochen | 4 Wochen | 2 Wochen | 4 Wochen |
| 21 | 96,9 | 94,8 | 1,90 | 3,97 |
| 20 | 98,5 | 94,7 | 1,89 | 4,09 |

Wie die Ergebnisse zeigen, ist die Rezeptur stabil genug, sodass man den Gehalt an dem Konservierungsmittel Benzalkoniumchlorid, soferne ein solcher Gehalt überhaupt gewünscht ist, in einem gewissen Rahmen variieren kann, ohne dass die Stabilität der Rezeptur darunter wesentlich leidet.

### Untersuchung der Stabilität von Desmopressin in Gegenwart von p-Hydroxybenzoesäuremethylester als Konservierungsmittel:

Hiefür wurde die Rezeptur Nr. 22 hergestellt, welche anstelle von Benzalkoniumchlorid p-Hydroxybenzoesäuremethylester mit einem Gehalt von 0,2 % als Konservierungsmittel aufweist.

Die so hergestellte Lösung wurde 4 Wochen bei 65°C gelagert und nach 2 und 4 Wochen im Hinblick auf den Gehalt an Desmopressin und dessen Abbauprodukten (G2 und G3) - ausgedrückt als Flächensumme - analysiert.

Es ergaben sich folgende Werte:

| Rezeptur Nr. | Desmopressinacetat (% vom Sollwert) nach | | Summe der Abbauprodukte G2, G3 (% A/A, bezogen auf Desmopressinacetat) nach | |
|---|---|---|---|---|
| | 2 Wochen | 4 Wochen | 2 Wochen | 4 Wochen |
| 22 | 92,7 | 88,0 | 1,51 | 3,0 |

Die Ergebnisse zeigen im Vergleich zu den Ergebnissen für Benzalkoniumchlorid als Konservierungsmittel, dass, wenn letzteres, etwa aus Verträglichkeitsgründen, nicht einsetzbar ist, dieses Konservierungsmittel durch ein anderes Konservierungsmittel ersetzt werden kann.

### Berechnung der möglichen Laufzeit:

Wie früher erwähnt, beträgt die Geschwindigkeitskonstante für den Desmopressinabbau im erfindungsgemäßen Puffersystem (Rezepturen Nr. 1, 13) 2,98 • 10⁸s⁻¹ in Gegenwart von Benzalkoniumchlorid, unter Abwesenheit von Konservierungsmittel (Rezeptur 15) 3,07 • 10⁸ s⁻¹.

Die Geschwindigkeitskonstante der im US-Patent Nr. 5,482,931 beschriebenen Formulierung A beträgt 4,6 • 10⁸ s⁻¹. Somit ist die Abbaugeschwindigkeit von Desmopressin im Apfelsäuresystem um den Faktor 1,5 - unabhängig von der Anwesenheit bzw. Abwesenheit von Benzalkoniumchlorid - erniedrigt. Dies bedeutet, dass die Stabilität und somit die mögliche Laufzeit der erfindungsgemäßen Zubereitung im Vergleich zu einer Zubereitung nach der erwähnten Formulierung A um etwa 50 % erhöht ist.

Im Vergleich zur in der obigen Literaturstelle erwähnten Formulierung B ergibt sich: die Geschwindigkeitskonstante des Desmopressinabbaues ist für die Formulierung B 8,0 • 10⁸ s⁻¹. Somit ist im Apfelsäuresystem die Stabilität des Desmopressins um das 2,6fache - unabhängig von der Anwesenheit bzw. Abwesenheit von Benzalkoniumchlorid - höher als im Citrat-Phosphatsystem. Daraus resultiert sogar eine um mehr als das Doppelte höhere Laufzeit im erfindungsgemäßen Puffersystem.

Im Sinne der Erfindung kann Desmopressin in handelsüblicher Form eingesetzt werden, d.h. rein oder in Form seiner Salze, z.B. als Acetat. Desgleichen kann die Apfelsäure in handelsüblicher Form eingesetzt werden, d.h. rein oder in Form ihrer üblichen Salze, z.B. als Natrium-Salz. Da die erfindungsgemäße pharmazeutische Zubereitung flüssig ist, liegt die Apfelsäure stets in gelöster Form vor.

Wenngleich Wasser als Lösungsmittel die weitaus überwiegende Anwendungsform ist, so besteht die Möglichkeit, auch andere Lösungsmittel, insbesondere Alkohol oder Mischungen von Wasser mit anderen Lösungsmitteln, einzusetzen. Desgleichen kann die erfindungsgemäße Zubereitung Restlösungsmittel in geringen Mengen enthalten.

Die folgenden Beispiele illustrieren das Wesen der Erfindung:

### Beispiel 1:

Zur Herstellung eines Nasensprays ohne Konservierungsmittel zur Behandlung von antidiuretischen Störungen und Blutungskrankheiten werden in einem 5 l-Becherglas 4900 g Aqua ad. inj. vorgelegt und darin 45,58 g Natriumchlorid, 1,675 g Apfelsäure und 0,5 g Desmopressinacetat unter Rühren gelöst. Der pH-Wert wird mit 1 N NaOH auf pH 5 eingestellt. Es wird auf 5 l mit Aqua ad. inj. aufgefüllt und diese Lösung durch ein steriles Millipak-Filter unter aseptischen Bedingungen in Braunglasfläschchen der hydrolytischen Klasse I abgefüllt und mit sterilen Pumpenköpfen und entsprechenden Nasenadaptoren verschlossen.

Die Herstellung und Abfüllung erfolgt in pharmazeutischen Reinräumen unter aseptischen Bedingungen.

### Beispiel 2:

Zur Herstellung eines Nasensprays mit Benzalkoniumchlorid als Konservierungsmittel zur Behandlung von antidiuretischen Störungen und Blutungskrankhelten werden in einem 1 l-Becherglas 990 g Aqua ad. inj. vorgelegt und darin 9,115 g Natriumchlorid, 0,1 g Desmopressinacetat, 0,1 g Benzalkoniumchlorid und 0,335 g Apfelsäure gelöst. Der pH-Wert wird mit ca. 4,2 ml 1 N NaOH auf pH 5 eingestellt, auf 1 l aufgefüllt, durch Millipak-Filter, filtriert und in Braunglasflaschen abgefüllt und mit den Pumpenaufsätzen verschlossen.

Die Herstellung und Abfüllung erfolgt in pharmazeutischen Produktionsräumen unter keimarmen Bedingungen.

### Beispiel 3:

Niedrigkonzentrierter Sublingualspray zur Behandlung von antidiuretischen Störungen und Blutungskrankheiten wird wie folgt hergestellt: Es werden 9900 g Aqua ad. inj. in ein entsprechendes Becherglas vorgelegt und darin 4 g Desmopressinacetat, 1 g Benzalkoniumchlorid, 91,15 g Natriumchlorid und 3,35 g Apfelsäure gelöst, der pH auf 5,0 eingestellt und auf 10 l mit Aqua ad. inj. aufgefüllt. Nach der Filtration wird in 100 ml Braunglasflaschen abgefüllt und die Gefäße mit entsprechenden Kunststoffkappen verschlossen.

Die Herstellung und Abfüllung erfolgt in Pharmaräumen unter keimarmen Bedingungen.

### Beispiel 4:

Zur Herstellung eines hochdosierten Sublingualsprays zur Behandlung von antidiuretischen Störungen und Blutungskrankheiten werden 2 g Desmopressinacetat, 0,1 g Benzalkoniumchlorid, 9,115 g Natriumchlorid und 0,335 g Apfelsäure in 950 g Aqua ad. inj. gelöst, der pH auf 5,0 eingestellt und auf 1 l mit Aqua ad. inj. aufgefüllt. Die Abfüllung erfolgt unter keimarmen Bedingungen in 50 ml Braunglasflaschen mit entsprechenden Kunststoffkappen.

### Beispiel 5:

Zur Herstellung eines Sirups zur oralen Anwendung (mit p-Hydroxybenzoesäuremethylester als Konservierungsmittel) zur Behandlung von antidiuretischen Störungen und Blutungskrankheiten werden 100 g Sorbit, 1,5 g SaccharinNatrium und 1,675 g Apfelsäure unter Rühren in 4,5l Aqua purificata gelöst. Daraufhin werden 100 mg Desmopressinacetat und 10 g p-Hydroxybenzoesäuremethylester (vorgelöst in heißem Wasser) in die Lösung eingerührt und nach Einstellung des pH-Wertes auf 5,0 mit Wasser auf 5 l aufgefüllt. Die Abfüllung erfolgt in 100 ml-Braunglasflaschen der hydrolytischen Klasse II.

Die Herstellung erfolgt in keimarmen Pharmaproduktionsräumen.

### Beispiel 6:

Zur Herstellung eines Sirups zur oralen Anwendung (mit p-Hydroxybenzoesäuremethylester und p-Hydroxybenzoesäurepropylester als Konservierungsmittel) zur Behandlung von antidiuretischen Störungen und Blutungskrankheiten werden 60 g Sorbit, 0,9 g SaccharinNatrium, 60 mg Desmopressinacetat und 1,005 g Apfelsäure in 2,7 l Aqua purificata gelöst und daraufhin 5,4 g p-Hydroxybenzoesäuremethylester und 0,6 g p-Hydroxybenzoesäurepropylester (vorgelöst in heißem Wasser) zugegeben, der pH-Wert auf 5,0 eingestellt, auf 3,0l mit Aqua purificata aufgefüllt und unter keimarmen Bedingungen in entsprechenden Braunglasflaschen mit Kunststoffverschlüssen abgefüllt.

Es hat sich gezeigt, dass eine Änderung des pH-Wertes stattfinden kann, wenn die Glasqualität der Glasgefäße, in welchen die Abfüllung erfolgt, nicht ausreicht. Aus diesem Grund ist es im Rahmen der Erfindung bei der Herstellung eines eine erfingungsgemäße Zubereitung enthaltenden Pharmazeutikums zweckmäßig, die Abfüllung in Glasgefäße der hydrolytischen Klasse I oder II durchzuführen, insbesondere dann, wenn die Apfelsäurekonzentration der verwendeten Lösung hoch ist.

Die in den vorangegangenen Beispielen angeführten Gehalte an Konservierungsmittel sind rein beispielsweise zu sehen. Es hat sich durch Versuche erwiesen, dass der Gehalt der Zubereitung an Benzalkoniumchlorid vorteilhaft zwischen 0,05 bis 0,20 mg/ml liegen kann. Desgleichen ist es im Rahmen der Erfindung durchaus möglich, dass der Gehalt an Konservierungsmittel Hydroxybenzoesäuremehtylester 1 bis 2,5 mg/ml beträgt. Besonders günstige Werte haben sich im Bereich von 1 bis 2 mg/ml ergeben. Der Konservierungsmittelgehalt an p-Hydroxybenzoesäuremethylester kann mit einem Gehalt an p-Hydroxybenzoesäurepropylester kombiniert sein, wobei letzterer Gehalt zweckmäßig zwischen 0 und 0,2 mg/ml liegt, vorzugsweise zwischen 0,1 und 0,2 mg/ml.

Der Wirkstoff (Desmopressin) wird zweckmäßig in niedriger Konzentration eingesetzt, etwa in Konzentrationen von 0,005 bis 2 mg/ml. Für Pharmazeutika, die zur oralen Anwendung gedacht sind, hat sich ein Desmopressingehalt von 0,005 bis 0,04 mg/ml als zweckmäßig erwiesen. Für Pharmazeutika zur nasalen Anwendung liegt der Gehalt in der Regel höher, etwa 0,02 bis 2,0 mg/ml, vorzugsweise 0,08 bis 1,0 mg/ml. Für sublingual anzuwendende Pharmazeutika ist hingegen der Desmopressingehalt in der Regel höher, etwa 0,4 bis 2,0 mg/ml.

## Patentansprüche

1. Stabile, nasal, oral oder sublingual anwendbare pharmazeutische Zubereitung zur Anwendung am Patienten, in Form einer flüssigen Lösung von Desmopressin als aktivem Wirkstoff, wobei diese Lösung ein Osmotikum und einen Puffer enthält, welcher den pH-Wert im Bereich 4 bis 6 hält, **dadurch gekennzeichnet, dass** zur Stabilisierung des Desmopressins der Puffer Apfelsäure ist.

2. Zubereitung nach Anspruch 1, **dadurch gekennzeichnet, dass** der pH-Wert bei etwa 5 liegt.

3. Zubereitung nach Anspruch 1 oder 2, **dadurch gekennzeichnet, dass** das Desmopressin in wässeriger Lösung vorliegt.

4. Zubereitung nach Anspruch 1, 2 oder 3, **dadurch gekennzeichnet, dass** der Apfelsäurepuffer in einer Konzentration von 1 bis 5 mM, insbesondere etwa 2,5 mM vorliegt.

5. Zubereitung nach einem der Ansprüche 1 bis 4, **dadurch gekennzeichnet, dass** die Apfelsäure als Racemat vorliegt.

6. Zubereitung nach einem der Ansprüche 1 bis 5, **dadurch gekennzeichnet, dass** das Desmopressin in einer Konzentration von 0,005 bis 2 mg/ml vorliegt.

7. Zubereitung nach Anspruch 6, **dadurch gekennzeichnet, dass** für eine zur oralen Anwendung bestimmte Zubereitung das Desmopressin in der Konzentration 0,005 bis 0,04 mg/ml vorliegt.

8. Zubereitung nach Anspruch 6, **dadurch gekennzeichnet, dass** für eine zur nasalen Anwendung bestimmte Zubereitung das Desmopressin in der Konzentration 0,02 bis 2,0 mg/ml, vorzugsweise 0,08 bis 1,0 mg/ml, insbesondere 0,1 mg/ml, vorliegt.

9. Zubereitung nach Anspruch 6, **dadurch gekennzeichnet, dass** für eine zur sublingualen Anwendung bestimmte Zubereitung das Desmopressin in der Konzentration 0,4 bis 2,0 mg/ml vorliegt.

10. Zubereitung nach einem der Ansprüche 1 bis 9, **dadurch gekennzeichnet, dass** NaCI zur Einstellung des osmotischen Druckes eingesetzt ist.

11. Zubereitung nach einem der Ansprüche 1 bis 10, **dadurch gekennzeichnet, dass** zusätzlich zur Apfelsäure ein weiterer Puffer eingesetzt ist, z.B. Acetat/Essigsäure.

12. Zubereitung nach einem der Ansprüche 1 bis 11, **dadurch gekennzeichnet, dass** sie frei ist von Konservierungsmitteln.

13. Zubereitung nach einem der Ansprüche 1 bis 11, **dadurch gekennzeichnet, dass** die Zubereitung 0,05 bis 0,20 mg/ml Benzalkoniumchlorid enthält.

14. Zubereitung nach einem der Ansprüche 1 bis 11, **dadurch gekennzeichnet, dass** die Zubereitung 1 bis 2,5 mg/ml, vorzugsweise 1 bis 2 mg/ml, inbesondere 2 mg/ml, p-Hydroxybenzoesäuremethylester enthält, gegebenenfalls in Kombination mit bis zu 0,2 mg/ml, vorzugsweise mit 0,1 bis 0,2 mg/ml, insbesondere mit 0,15 bis 0,2 mg/ml, p-Hydroxybenzoesäurepropylester.

15. Zubereitung nach einem der Ansprüche 1 bis 14, **dadurch gekennzeichnet, dass** sie 0,1 mg/ml Desmopressinacetat, gelöst in Wasser, enthält sowie DL-Apfelsäure in einer Konzentration von 2,5 mM und NaCI als Osmotikum, sowie gegebenenfalls 0,10 mg/ml Benzalkoniumchlorid als Konservierungsmittel, wobei der pH-Wert der Zubereitung bei etwa 5 gehalten ist.

16. Zubereitung nach einem der Ansprüche 1 bis 15, **dadurch gekennzeichnet, dass** die Apfelsäure in Form eines Salzes, z.B. des Natrium-Salzes, in gelöstem Zustand vorliegt.

17. Verfahren zur Herstellung eines eine Zubereitung nach einem der Ansprüche 1 bis 16 enthaltenden Pharmazeutikums, wobei die Zubereitung in Glasgefäße der hydrolytischen Klasse I oder II abgefüllt wird.

18. Nasal anwendbares Pharmazeutikum, enthaltend eine Zubereitung nach einem der Ansprüche 1 bis 16.

19. Oral anwendbares Pharmazeutikum, enthaltend eine Zubereitung nach einem der Ansprüche 1 bis 16.

20. Sublingual anwendbares Pharmazeutikum, enthaltend eine Zubereitung nach einem der Ansprüche 1 bis 16.

21. Verwendung einer pharmazeutischen Zubereitung nach einem der Ansprüche 1 bis 16 zur Herstellung eines Arzneimittels zur Behandlung eines an antidiuretischen Störungen, wie Enuresis nocturma oder Diabetes insipidus, leidenden Patienten.

22. Verwendung einer pharmazeutischen Zubereitung nach einem der Ansprüche 1 bis 16 zur Herstellung eines Arzneimittels zur Behandlung eines an Blutungskrankheiten, wie Hämophilie A, Willebrand-Jürgens-Syndrom oder postoperativen Blutungen, leidenden Patienten.

## Claims

1. A stable, nasally, orally or sublingually administrable pharmaceutical preparation for administration to patients in the form of a liquid solution of desmopressin as the active ingredient, wherein said solution contains an osmotic agent and a buffer which maintains the pH value in the range from 4 to 6, **characterised in that** the buffer for stabilising the desmopressin is malic acid.

2. A preparation according to claim 1, **characterised in that** the pH value is at approx. 5.

3. A preparation according to claim 1 or 2, **characterised in that** the desmopressin is present in an aqueous solution.

4. A preparation according to claim 1, 2 or 3, **characterised in that** the malic acid buffer is present in a concentration of 1 to 5 mM, in particular of approx. 2.5 mM.

5. A preparation according to one of claims 1 to 4, **characterised in that** the malic acid is present as a racemate.

6. A preparation according to one of claims 1 to 5, **characterised in that** the desmopressin is present in a concentration of 0.005 to 2 mg/ml.

7. A preparation according to claim 6, **characterised in that**, in a preparation intended for oral administration, the desmopressin is present in a concentration of 0.005 to 0.04 mg/ml.

8. A preparation according to claim 6, **characterised in that**, in a preparation intended for nasal administration, the desmopressin is present in a concentration of 0.02 to 2.0 mg/ml, preferably of 0.08 to 1.0 mg/ml, in particular of 0.1 mg/ml.

9. A preparation according to claim 6, **characterised in that**, in a preparation intended for sublingual administration, the desmopressin is present in a concentration of 0.4 to 2.0 mg/ml.

10. A preparation according to one of claims 1 to 9, **characterised in that** NaCl is used to establish the osmotic pressure.

11. A preparation according to one of claims 1 to 10, **characterised in that**, in addition to the malic acid, a further buffer is used, for example acetate/acetic acid.

12. A preparation according to one of claims 1 to 11, **characterised in that** it contains no preservatives.

13. A preparation according to one of claims 1 to 11, **characterised in that** the preparation contains 0.05 to 0.20 mg/ml of benzalkonium chloride.

14. A preparation according to one of claims 1 to 11, **characterised in that** the preparation contains 1 to 2.5 mg/ml, preferably 1 to 2 mg/ml, in particular 2 mg/ml, of p-hydroxybenzoic acid methyl ester, optionally in combination with up to 0.2 mg/ml, preferably with 0.1 to 0.2 mg/ml, in particular with 0.15 to 0.2 mg/ml, of p-hydroxybenzoic acid propyl ester.

15. A preparation according to one of claims 1 to 14, **characterised in that** it contains 0.1 mg/ml of desmopressin acetate dissolved in water, together with DL-malic acid in a concentration of 2.5 mM and NaCl as osmotic agent, optionally together with 0.10 mg/ml of benzalkonium chloride as preservative, wherein the pH value of the preparation is maintained at approx. 5.

16. A preparation according to one of claims 1 to 15, **characterised in that** the malic acid is present in the dissolved state in the form of a salt, for example the sodium salt.

17. A process for the manufacture of a pharmaceutical containing a preparation according to one of claims 1 to 16, wherein the preparation is packaged in glass containers of hydrolytic class I or II.

18. A nasally administrable pharmaceutical containing a preparation according to one of claims 1 to 16.

19. An orally administrable pharmaceutical containing a preparation according to one of claims 1 to 16.

20. A sublingually administrable pharmaceutical containing a preparation according to one of claims 1 to 16.

21. Use of a pharmaceutical preparation according to one of claims 1 to 16 for the manufacture of a medicament for the treatment of a patient suffering from antidiuretic disorders, such as nocturnal enuresis or diabetes insipidus.

22. Use of a pharmaceutical preparation according to one of claims 1 to 16 for the manufacture of a medicament for the treatment of a patient suffering from bleeding disorders, such as haemophilia A, von Willebrand's disease, or postoperative bleeding.

## Revendications

1. Préparation pharmaceutique stable, pouvant être administrée au patient par voie nasale, orale ou sublinguale sous la forme d'une solution liquide de desmopressine en tant que principe actif, cette solution contenant un agent osmotique et une solution tampon qui maintient le pH entre 4 et 6, **caractérisée en ce que** le tampon stabilisant la desmopressine est l'acide malique.

2. Préparation selon la revendication 1, **caractérisée en ce que** le pH est de l'ordre de 5.

3. Préparation selon la revendication 1 ou 2, **caractérisée en ce que** la desmopressine se présente sous forme de solution aqueuse.

4. Préparation selon la revendication 1, 2 ou 3, **caractérisée en ce que** le tampon d'acide malique est présent à une concentration comprise entre 1 et 5 mM et en particulier égale à 2,5 mM environ.

5. Préparation selon l'une des revendications 1 à 4, **caractérisée en ce que** l'acide malique se présente sous forme racémique.

6. Préparation selon la revendication 1 à 5, **caractérisée en ce que** la desmopressine est présente à une concentration comprise entre 0,005 et 2 mg/ml.

7. Préparation selon la revendication 6, **caractérisée en ce que** pour une préparation administrée par voie orale, la desmopressine est présente à une concentration comprise entre 0,005 et 0,04 mg/ml.

8. Préparation selon la revendication 6, **caractérisée en ce que** pour une préparation administrée par voie nasale, la desmopressine est présente à une concentration comprise entre 0,02 et 2,0 mg/ml, de préférence 0,08 à 1,0 mg/ml, et en particulier égale à 0,1 mg/ml.

9. Préparation selon la revendication 6, **caractérisée en ce que** pour une préparation administrée par voie sublinguale, la desmopressine est présente à une concentration comprise entre 0,4 et 2,0 mg/ml.

10. Préparation selon l'une quelconque des revendications 1 à 9, **caractérisée en ce qu'**elle contient du NaCl pour régler la pression osmotique.

11. Préparation selon l'une quelconque des revendications 1 à 10, **caractérisée en ce qu'**elle contient, outre l'acide malique, un autre tampon, par exemple acétate/acide acétique.

12. Préparation selon l'une quelconque des revendications 1 à 11, **caractérisée en ce qu'**elle ne contient pas d'agent conservateur.

13. Préparation selon l'une quelconque des revendications 1 à 11, **caractérisée en ce qu'**elle contient 0,05 à 0,20 mg/ml de chlorure de benzalkonium.

14. Préparation selon l'une quelconque des revendications 1 à 11, **caractérisée en ce qu'**elle contient 1 à 2,5 mg/ml, de préférence 1 à 2 mg/ml, en particulier 2mg/ml d'ester méthylique de l'acide p-hydroxybenzoïque, éventuellement en combinaison avec jusqu'à 0,2 mg/ml, de préférence entre 0,1 et 0,2 mg/ml et en particulier entre 0,15 et 0,2 mg/ml d'ester propylique de l'acide p-hydroxybenzoïque.

15. Préparation selon l'une quelconque des revendications 1 à 14, **caractérisée en ce qu'**elle contient 0,1 mg/ml d'acétate de desmopressine, en solution dans l'eau, ainsi que de l'acide malique DL à une concentration de 2,5 mM et du NaCl en tant qu'agent osmotique, ainsi qu'éventuellement 0,10 mg/ml de chlorure de benzalkonium en tant qu'agent conservateur, le pH de la préparation étant maintenu autour de 5.

16. Préparation selon l'une quelconque des revendications 1 à 15, **caractérisée en ce que** l'acide malique est présent sous forme de sel, par exemple de sel de sodium, en solution.

17. Procédé de production d'un produit pharmaceutique contenant une préparation selon l'une quelconque des revendications 1 à 16, la préparation étant conditionnée dans des contenants en verre de classe hydrolytique I ou II.

18. Produit pharmaceutique administré par voie nasale, contenant une préparation selon l'une quelconque des revendications 1 à 16.

19. Produit pharmaceutique administré par voie orale, contenant une préparation selon l'une quelconque des revendications 1 à 16.

20. Produit pharmaceutique administré par voie sublinguale, contenant une préparation selon l'une quelconque des revendications 1 à 16.

21. Utilisation d'une préparation pharmaceutique selon l'une quelconque des revendications 1 à 16 pour produire un médicament de traitement d'un patient souffrant de dysfonctionnements du système antidiurétique comme l'énurésie nocturne ou le diabète insipide.

22. Utilisation d'une préparation pharmaceutique selon l'une quelconque des revendications 1 à 16 pour produire un médicament de traitement d'un patient souffrant de troubles hémorragiques comme l'hémophilie A, le syndrome de von Willebrand-Jürgens ou des hémorragies post-opératoires.
